# DEMANDE DE BREVET EUROPEEN

(11) **EP 4 810 147 A1**
(43) Date de publication de la demande: **23.09.2026**
(21) Numéro de dépôt: 26158332.2
(22) Date de dépôt: 12.02.2026
(51) Int. Cl.: B01F 23/235, B01F 25/451, B01F 23/41, B01F 31/65, B01F 35/42

(54) **DISPOSITIF DE PRÉPARATION D'UNE MOUSSE SCLÉROSANTE DESTINÉE À ÊTRE INJECTÉE DANS UN CORPS D'UN PATIENT**

(30) Priorité: 21.03.2025 FR 2502956
(71) Demandeur: Galbin, Pierre, 22370 Pleneuf Val André (FR)
(72) Inventeur: Galbin, Pierre, 22370 Pleneuf Val André (FR)
(74) Mandataire: Vidon Brevets & Stratégie

(57) **Abrégé**

L'invention concerne un dispositif de préparation d'une mousse sclérosante à partir d'un sclérosant liquide et d'un volume d'air, ladite mousse sclérosante étant destinée à être injectée dans un corps d'un patient, ledit dispositif (1) comprenant : un châssis (10), un premier actionneur (21) disposé dans un premier bras (101), un deuxième actionneur (22) disposé dans un deuxième bras (102), un premier moteur (31), un deuxième moteur (32), le premier actionneur (21), le deuxième actionneur (22), le premier moteur (31) et le deuxième moteur (32) étant configurés de sorte que lorsqu'ils sont en mouvement un sclérosant liquide et un volume d'air se mélangent dans des seringues.

## Description

### Domaine technique

La présente invention se rapporte au domaine des dispositifs médicaux, et plus particulièrement à un dispositif de préparation d'une mouse sclérosante destinée à être injectée dans un corps d'un patient pour un traitement de varices par sclérothérapie.

### Art Antérieur

La sclérothérapie est une technique médicale qui consiste en l'ablation chimique de varices par injection intra-veineuse d'une substance sclérosante. A l'aide d'une aiguille, la substance sclérosante est injectée dans la veine à traiter sous la forme d'une mousse sclérosante ce qui provoque un durcissement de cette veine et sa disparition à terme.

L'utilisation de la mousse comme produit sclérosant est de plus en plus répandue. Par « mousse », on entend un mélange de produit sclérosant et d'air. Elle présente une forme compacte à l'injection avec des microbulles de taille homogène, de l'ordre de 100µm environ.

Un produit à injecter sous forme de mousse permet d'augmenter l'effet sclérosant pour obtenir un meilleur résultat en limitant la consommation de produit. Les propriétés physico-chimiques de la mousse, d'un point de vue viscosité et stabilité, permettent à celle-ci d'agir localement sur la zone à traiter. La forme compacte de la mousse évite une diffusion du produit sclérosant dans le réseau sanguin du patient et permet un contact direct avec la paroi interne de la varice.

Il est connu d'utiliser un procédé manuel pour obtenir de la mousse sclérosante. Dans un tel procédé, un praticien utilise deux seringues et un connecteur. Dans un premier temps, le praticien remplit partiellement la première seringue avec le sclérosant liquide. Il remplit également la deuxième seringue avec un volume d'air, puis il connecte les deux seringues via le connecteur. Dans des mouvements de va-et-vient des pistons des seringues, il mélange le sclérosant liquide et le volume d'air de sorte à obtenir une mousse sclérosante après plusieurs mouvements des pistons. Il recueille, enfin, la mousse sclérosante dans une seule seringue en vue d'une injection dans le corps du patient.

Un tel procédé est, cependant, contraignant pour le praticien. En effet, la génération des mouvements de va-et-vient nécessite des pressions manuelles répétées sur les pistons des seringues. Ces pressions peuvent créer à terme une fatigue, voire des troubles musculosquelettiques pour le praticien, notamment en cas d'injections répétées pour plusieurs patients successifs.

Il existe donc un besoin d'améliorer les solutions existantes en vue de rendre moins fastidieuse la fabrication d'une mousse sclérosante par un praticien.

### Résumé de l'invention

Un premier objet de l'invention concerne un dispositif de préparation d'une mousse sclérosante à partir d'un sclérosant liquide et d'un volume d'air, ladite mousse sclérosante étant destinée à être injectée dans un corps d'un patient, ledit dispositif comprenant :

- un châssis comportant une partie en forme de V comprenant un premier bras, un deuxième bras et une zone de jonction entre le premier bras et le deuxième bras, ledit premier bras étant adapté pour recevoir une première seringue, ledit deuxième bras étant adapté pour recevoir une deuxième seringue, la zone de jonction étant adaptée pour recevoir un connecteur disposé entre la première seringue et la deuxième seringue ;

- un premier actionneur disposé dans le premier bras, ledit premier actionneur étant destiné à agir sur la première seringue pour modifier un volume d'une première chambre de mélange de ladite première seringue ;

- un deuxième actionneur disposé dans le deuxième bras, ledit deuxième actionneur étant destiné à agir sur la deuxième seringue pour modifier un volume d'une deuxième chambre de mélange de ladite deuxième seringue ;

- un premier moteur, disposé à une extrémité du premier bras opposée à la zone de jonction, ledit premier moteur étant apte à contrôler le mouvement du premier actionneur ;

- un deuxième moteur, disposé à une extrémité du deuxième bras opposée à la zone de jonction, ledit deuxième moteur étant apte à contrôler le mouvement du deuxième actionneur ;

- le premier actionneur, le deuxième actionneur, le premier moteur et le deuxième moteur étant configurés de sorte que lorsqu'ils sont en mouvement, le volume de la première chambre de mélange de la première seringue et le volume de la deuxième chambre de mélange de la deuxième seringue sont modifiés de manière alternée de sorte à permettre un mélange du sclérosant liquide et du volume d'air dans la première seringue et dans la deuxième seringue via le connecteur.

L'invention permet ainsi d'automatiser la fabrication de la mousse sclérosante d'une manière simple et pratique, ce qui limite les risques de fatigue pour le praticien. L'intervention de celui-ci se limite au remplissage de chaque seringue par du sclérosant liquide ou de l'air et le positionnement des seringues dans le dispositif. A la fin du processus de mélangeage, le praticien récupère la mousse sclérosante dans une seule seringue.

En outre, l'utilisation d'un tel dispositif mécanisé permet d'améliorer l'uniformisation de la mousse sclérosante obtenue entre deux préparations.

Dans une variante de réalisation, la partie en forme de V présente un angle interne mesuré entre le premier bras et le deuxième bras compris entre 45 degrés et 180 degrés.

Dans une variante de réalisation, la partie en forme de V est inclinée par rapport à un plan horizontal.

Dans une variante de réalisation, le connecteur est un robinet à trois voies.

Dans une variante de réalisation, le premier moteur et le deuxième moteur comprennent respectivement :
- un corps de moteur ;
- un arbre moteur apte à être en rotation par rapport au corps de moteur, ledit arbre moteur présentant une extrémité ;
- une première plaque plate allongée présentant une première extrémité proximale et une première extrémité distale, ladite première extrémité proximale étant solidaire de l'extrémité de l'arbre moteur ;
- une deuxième plaque plate allongée présentant une deuxième extrémité proximale et une deuxième extrémité distale, ladite deuxième extrémité proximale étant fixée en liaison pivot avec la première extrémité distale de la première plaque plate allongée via une première liaison pivot, ladite deuxième extrémité distale étant fixée via une deuxième liaison pivot avec, respectivement, le premier actionneur ou le deuxième actionneur de sorte à mettre en mouvement ledit premier actionneur ou ledit deuxième actionneur lors de la rotation de l'arbre moteur.

Dans une variante de réalisation, la première plaque plate allongée présente une première longueur entre la première extrémité proximale et la première extrémité distale et la deuxième plaque plate allongée présente une deuxième longueur entre la deuxième extrémité proximale et la deuxième extrémité distale, ladite deuxième longueur étant supérieure à ladite première longueur.

Dans une variante de réalisation, le dispositif comprend un potentiomètre pour gérer la vitesse de déplacement du premier actionneur et du deuxième actionneur.

Un autre objet de l'invention concerne un système de préparation d'une mousse sclérosante à partir d'un sclérosant liquide et d'un volume d'air, ladite mousse sclérosante étant destinée à être injectée dans un corps d'un patient. Ce système comprend :
- un dispositif de préparation de la mousse sclérosante selon l'invention ;
- un interrupteur d'alimentation pour interrompre une alimentation électrique du dispositif de préparation ;
- un détecteur d'un paramètre de la mousse sclérosante en préparation ;
- des moyens d'analyse, lesdits moyens d'analyse étant aptes à déterminer si le paramètre détecté est supérieur à un certain seuil et à activer l'interrupteur d'alimentation en conséquence.

Dans une variante de réalisation, les moyens d'analyse comprennent un module d'intelligence artificielle.

Un autre objet de l'invention concerne une mousse sclérosante destinée à être injectée dans un corps d'un patient, ladite mousse sclérosante étant préparée à l'aide d'un dispositif de préparation selon l'invention.

### Description des figures

D'autres caractéristiques et avantages de l'invention apparaîtront au cours de la lecture de la description détaillée qui va suivre pour la compréhension de laquelle on se reportera aux dessins annexés dans lesquels :
La figure 1 illustre un dispositif de préparation d'une mousse sclérosante selon l'invention, dans une vue avant ;
La figure 2 illustre le dispositif de préparation de la figure 1, dans une vue arrière ;
La figure 3 illustre schématiquement un actionneur du dispositif de préparation de la figure 1 ;
La figure 4 illustre schématiquement un moteur du dispositif de préparation de la figure 1 ;
La figure 5 illustre schématiquement le dispositif de préparation de la figure 1 dans une première position de fonctionnement ;
La figure 6 illustre schématiquement le dispositif de préparation de la figure 1 dans une seconde position de fonctionnement ;
La figure 7 illustre schématiquement le dispositif de préparation de la figure 1 dans une troisième position de fonctionnement ;
La figure 8 illustre schématiquement le dispositif de préparation de la figure 1 dans une quatrième position de fonctionnement ;
La figure 9 illustre une première seringue et une deuxième seringue reliées par un connecteur, l'ensemble étant apte à être positionné dans le dispositif de préparation de la figure 1 ;
La figure 10 illustre une première étape d'un procédé de préparation de la mousse à l'aide du dispositif de la figure 1 ;
La figure 11 illustre une deuxième étape du procédé de préparation de la mousse à l'aide du dispositif de la figure 1 ;
La figure 12 illustre une troisième étape du procédé de préparation de la mousse à l'aide du dispositif de la figure 1 ;
La figure 13 illustre une quatrième étape du procédé de préparation de la mousse à l'aide du dispositif de la figure 1 ;
La figure 14 illustre une mousse sclérosante obtenue à partir des étapes du procédé de préparation des figures 10 à 13 ;
La figure 15 illustre un système de préparation d'une mousse sclérosante comportant le dispositif de préparation de la figure 1.

### Description de modes de réalisation

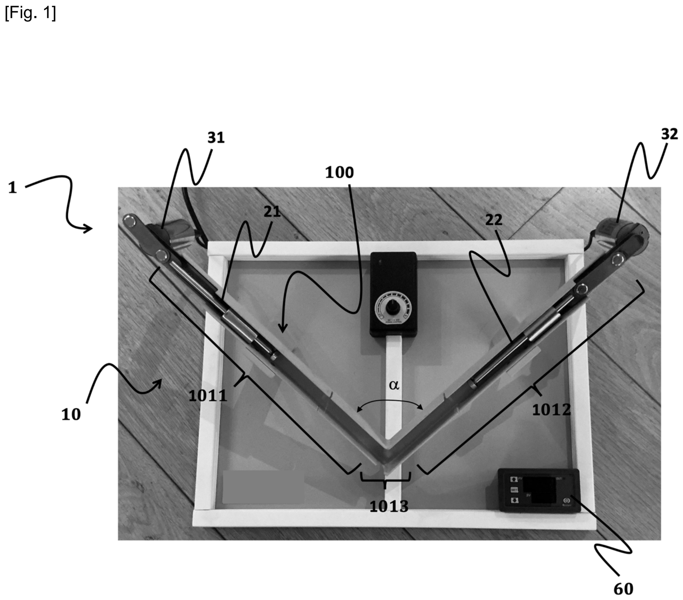

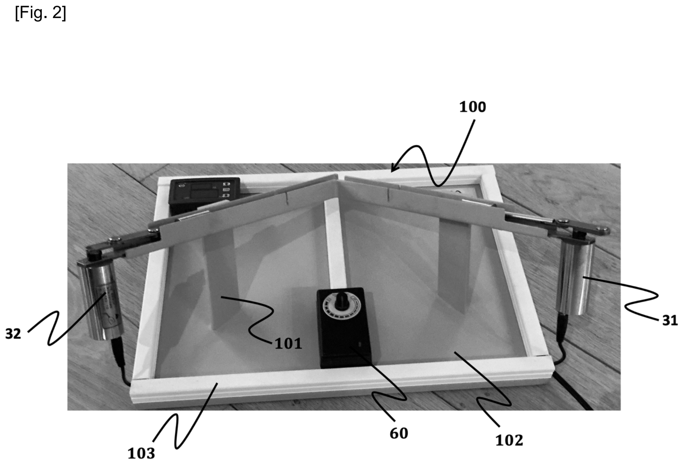

La figure 1 et la figure 2 illustrent un dispositif 1 de préparation d'une mousse sclérosante selon l'invention.

Ce dispositif 1 comprend :
- un châssis 10 ;
- un premier actionneur 21 ;
- un deuxième actionneur 22 ;
- un premier moteur 31 ;
- un deuxième moteur 32 ;
- un potentiomètre 60.

Le châssis 10 comprend :
- une partie 100 en forme de V ;
- des supports 101 ;
- une plaque 102 ;
- des renforts 103.

La partie 100 en forme de V comprend :
- un premier bras 1011 ;
- un deuxième bras 1012 ;
- une zone de jonction 1013 entre le premier bras 1011 et le deuxième bras 1012.

Le premier bras 1011, le deuxième bras 1012 et la zone de jonction 1013 sont creux et ouverts, de sorte qu'il est possible d'y insérer des éléments pour la préparation de la mousse.

Le premier bras 1011 est ainsi adapté pour recevoir une première seringue 41.

Le deuxième bras 1012 est adapté pour recevoir une deuxième seringue 42.

La zone de jonction 1013 est adaptée pour recevoir un connecteur 50.

La partie 100 en forme de V présente un angle interne α mesuré entre le premier bras 1011 et le deuxième bras 1012. Cet angle interne α est compris entre 45 degrés et 180 degrés.

Préférentiellement, l'angle interne α est compris entre 80 degrés et 100 degrés.

Préférentiellement, l'angle interne α est égal à 90 degrés.

Les supports 101 sont adaptés pour supporter la partie 100 en forme de V. Dans l'exemple de la figure 2, ces supports 101 se présentent sous la forme de deux mâts verticaux.

La plaque 102 est adaptée pour recevoir les supports 101. Cette plaque 102 est, ici, horizontale. Les supports 101 sont agencés de sorte à incliner la partie 100 en forme de V. La partie 100 en forme de V forme, ainsi, un angle d'inclinaison avec la plaque 102 horizontale. Cet angle d'inclinaison est compris entre 20 degrés et 40 degrés. Préférentiellement, l'angle d'inclinaison est égal à 30 degrés.

Les renforts 103 sont disposés sur le pourtour de la plaque 102. Ces renforts 103 permettent de rigidifier l'ensemble du châssis 10.

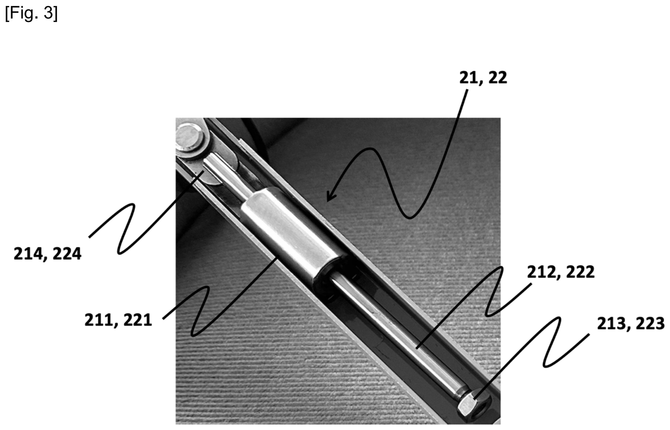

La figure 3 illustre schématiquement un actionneur du dispositif 1 de préparation de la mousse. La description de cette figure 3 s'applique au premier actionneur 21 et au deuxième actionneur 22. Pour décrire les différents éléments constitutifs de ces actionneurs 21, 22, il est utilisé des références 21X pour le premier actionneur 21 et des références 22X pour le deuxième actionneur.

L'actionneur 21, 22 de la figure 3 comprend :
- un élément de guidage 211, 221 ;
- une tige 212, 222 ;
- une tête 213, 223 ;
- une masselotte 214, 224.

L'élément de guidage 211, 221 se présente sous la forme d'un cylindre comportant une cavité dans sa partie centrale débouchant de chaque côté dudit cylindre. L'élément de guidage 211, 221 permet de maintenir un bon guidage de l'actionneur 21, 22 à l'intérieur du premier bras 1011, respectivement du deuxième bras 1012, de sorte à éviter tout risque d'arc-boutement.

La tige 212, 222 s'étend dans la longueur du premier bras 1011, respectivement du deuxième bras 1012. Elle est adaptée pour coulisser à l'intérieur de l'élément de guidage 211, 221 via la cavité.

La tête 213, 223 est disposée à une extrémité de la tige 212, 222. Elle est destinée à venir en contact avec un piston d'une seringue 41, 42. Cette tête 213, 223 se présente, ici, sous la forme d'un écrou directement vissé sur la tige 212, 222.

La masselotte 214, 224 est disposée à une extrémité de la tige 212, 222 à l'opposé de la tête 213, 223. Cette masselotte est ici solidaire de la tige 212, 222. Elle permet à la tige 212, 222 d'être reliée au moteur 31, 32.

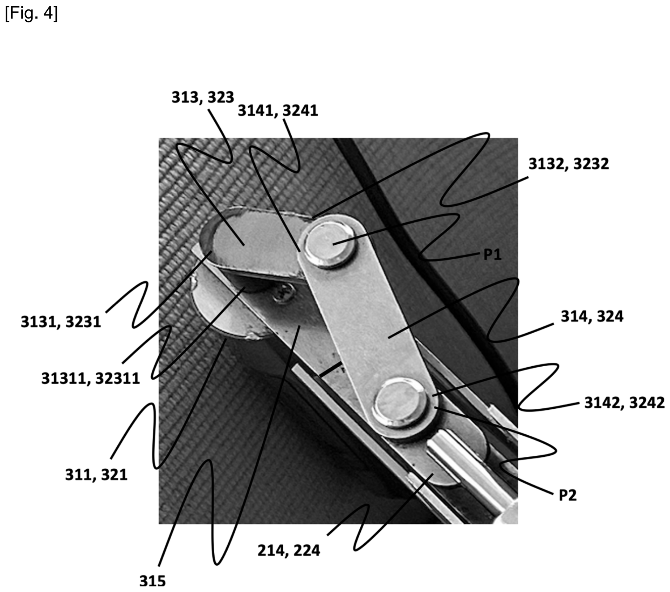

La figure 4 illustre schématiquement un moteur 31, 32 du dispositif 1 de préparation de la mousse. La description de cette figure 4 s'applique au premier moteur 31 et au deuxième moteur 32. Pour décrire les différents éléments constitutifs de ces moteurs 31, 32, il est utilisé des références 31X pour le premier moteur 31 et des références 32X pour le deuxième moteur.

Le moteur 31, 32 de la figure 4 comprend :
- un corps de moteur 311, 321 ;
- un arbre moteur (non visible sur la figure 4) ;
- une première plaque 313, 323 ;
- une deuxième plaque 314, 324 ;
- une plaque support 315.

Le corps de moteur 311, 321 se présente sous la forme d'une pièce cylindrique qui s'étend selon une direction verticale, comme il est plus particulièrement visible à la figure 2.

L'arbre moteur est disposé à l'intérieur du corps du moteur 311, 321. Il est apte à tourner par rapport audit corps 311, 321. Il présente une extrémité (non visible sur la figure 4) qui permet de transmettre un couple de rotation. Cette extrémité est, par exemple, un arbre cannelé.

La première plaque 313, 323 se présente sous la forme d'une plaque plate allongée. Elle comporte une première extrémité proximale 3131, 3231 et une première extrémité distale 3132, 3232. La première extrémité proximale 3131, 3231 comporte un embout 31311, 32311 adapté pour venir se loger sur la partie cannelée de l'extrémité de l'arbre moteur. Cet embout 31311, 32311 comporte des cannelures complémentaires à l'extrémité de l'arbre moteur, pour une transmission du couple moteur. La première extrémité distale 3132, 3232 est disposée à l'opposé de la première extrémité proximale 3131, 3231 sur la première plaque 313, 323.

La deuxième plaque 314, 324 se présente sous la forme d'une plaque plate allongée. Elle comporte une deuxième extrémité proximale 3141, 3241 et une deuxième extrémité distale 3142, 3242. La deuxième extrémité proximale 3141, 3241 est fixée avec la première extrémité distale de la première plaque allongée 313, 323 via une première liaison pivot P1. La deuxième extrémité distale 3142, 3242 est fixée avec la masselotte 214, 224 et donc l'actionneur 21, 22 via une deuxième liaison pivot P2.

On notera que la deuxième plaque 314, 324 présente une longueur supérieure à la première plaque 313, 323.

La plaque support 315 est fixée au corps de moteur 311, 312. Elle s'étend dans le prolongement de la masselotte 214, 224 de l'actionneur 21, 22, de sorte que cette masselotte 214, 224 est adaptée pour glisser sur la plaque support 315 lors du mouvement de l'arbre moteur, de la première plaque 313, 323 et de la deuxième plaque 314, 324.

Le moteur 31, 32 est alimenté pas une alimentation électrique (non visible sur la figure 4).

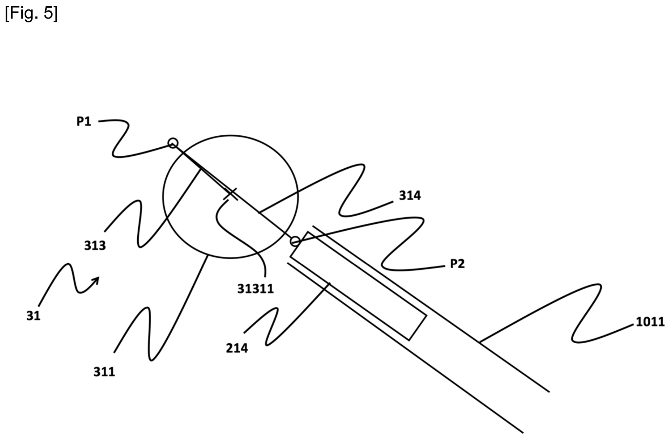

Les figures 5 à 8 illustrent une cinématique de différents éléments du premier moteur 31 et de la masselotte 214 associés au premier bras 1011. Cette description s'appliquera également aux éléments du deuxième moteur 32 associés au deuxième bras 1012.

Dans un premier état illustré à la figure 5, la deuxième plaque allongée 314 vient recouvrir la première plaque allongée 313. La première liaison pivot P1, l'embout 31311 et la deuxième liaison pivot P2 sont globalement alignés. La masselotte 214 est, ici, positionnée dans une position la plus proche du premier moteur 31.

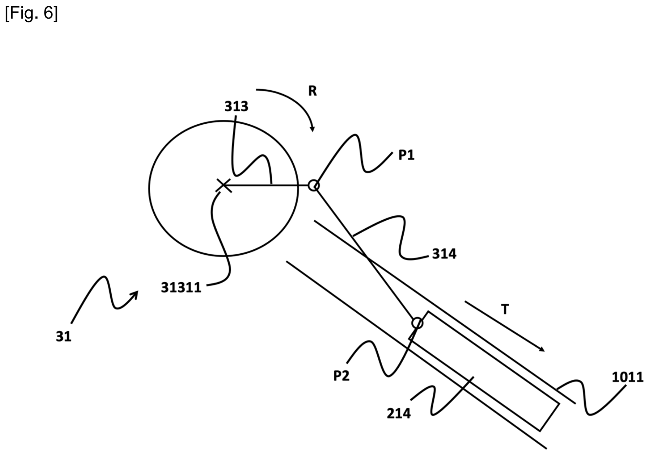

Dans un deuxième état illustré à la figure 6, sous l'action de l'arbre moteur, l'embout 31311 vient entrainer en rotation R dans un sens horaire la première plaque allongée 313. Ce mouvement entraîne la masselotte 214 en translation T dans le premier bras 1011. La masselotte 214 s'éloigne alors du premier moteur 31 dans une position intermédiaire. On notera que la deuxième plaque allongée 314 est au-dessus des bords du premier bras 1011, ce qui lui permet d'agir directement sur la masselotte 214 via la deuxième liaison pivot P2.

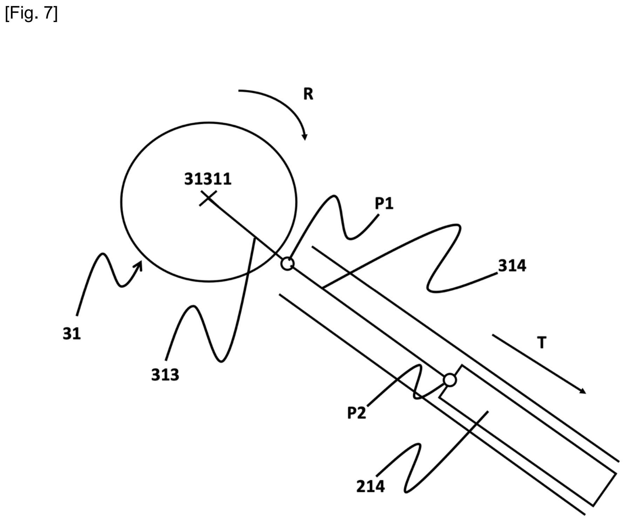

Dans un troisième état illustré à la figure 7, l'embout 31311, la première liaison pivot P1 et la deuxième liaison pivot P2 sont alignés. La masselotte 214 est alors dans une position la plus éloignée du premier moteur 31.

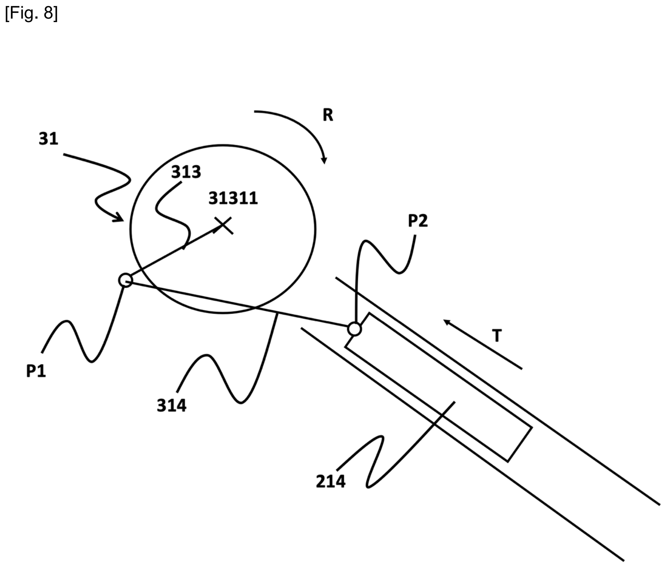

Dans un quatrième état illustré à la figure 8, la première plaque allongée 313 revient vers sa position initiale. La première plaque allongée 312, la deuxième plaque allongée 314, la première liaison pivot P1 et la deuxième liaison pivot P2 agissent alors pour ramener la masselotte 214 vers le premier moteur 31.

On notera que la vitesse de rotation de l'arbre moteur, et donc la vitesse de déplacement de la masselotte 214, est réglée à l'aide du potentiomètre 60.

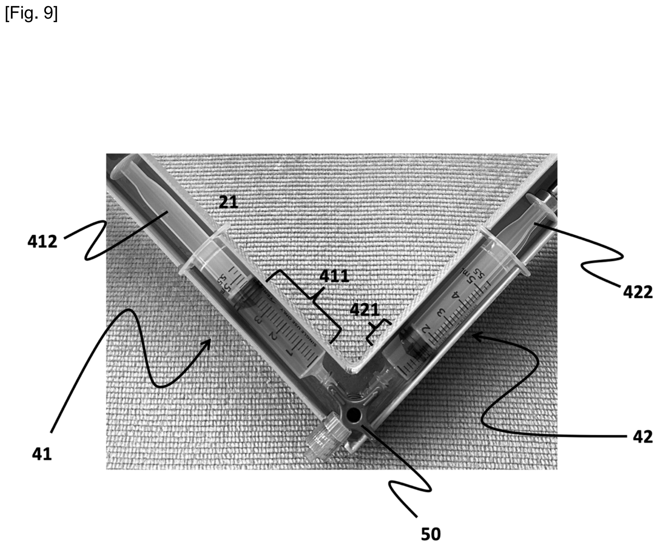

La figure 9 illustre la première seringue 41 logée dans le premier bras 1011 et la seconde seringue 42 logée dans le deuxième bras 1012.

La première seringue 41 comprend une première chambre de mélange 411. Cette chambre 411 est destinée à recevoir le sclérosant liquide et/ou le volume d'air. Le volume de la première chambre de mélange 411 est modifié par un premier piston 412. C'est sur ce premier piston 412 que vient agir la tête 213 de l'actionneur 21.

La deuxième seringue 42 comprend une deuxième chambre de mélange 421. Cette chambre 421 est destinée à recevoir le sclérosant liquide et/ou le volume d'air. Le volume de la deuxième chambre de mélange 421 est modifié par un deuxième piston 422. C'est sur ce deuxième piston 422 que vient agir la tête 223 de l'actionneur 22.

Le connecteur 50 vient relier la première seringue 41 et la deuxième seringue 42. Il permet le passage du sclérosant liquide et/ou du volume d'air entre les deux chambres de mélange 411, 421.

Le sclérosant liquide est, par exemple le produit Aetoxisclérol concentré à niveau compris entre 1% et 3%.

Dans le mode de réalisation de la figure 9, le connecteur 50 est un robinet à trois voies. Préférentiellement, le connecteur 50 est un robinet à trois voies type B Braun Discofix^{®}.

En variante, le connecteur 50 est une tubulaire à deux voies.

Dans une autre variante, le connecteur 50 est droit et souple de type Kreussler^{®}.

Dans une autre variante, le connecteur 50 est droit et rigide de type Luer lock^{®}.

On notera que la première chambre de mélange 411 et la deuxième chambre de mélange 421 ont, par exemple, une capacité maximale égale à 5 millilitres.

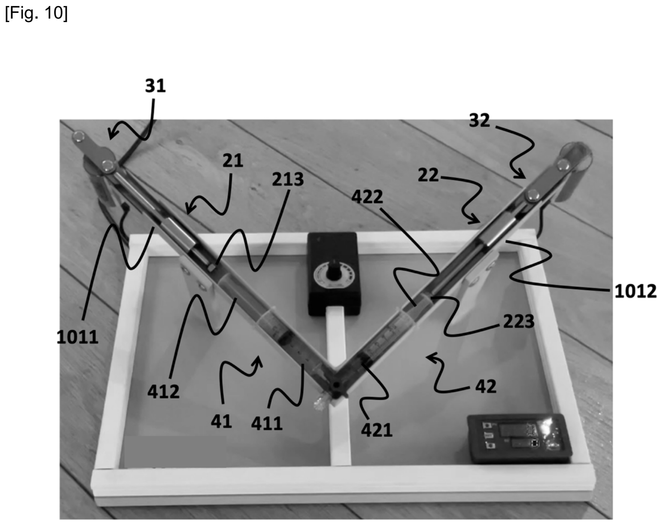

Les figures 10 à 13 illustrent différentes étapes d'un procédé de préparation de la mousse à l'aide du dispositif de préparation 10.

Dans une première étape illustrée à la figure 10, la première seringue 41 est disposée dans le premier bras 1011.

De la même manière, la deuxième seringue 42 est disposée dans le deuxième bras 1012.

Dans cette étape, les deux seringues 41, 42 ne sont pas dans la même configuration. Ainsi, dans la première seringue 41, le premier piston 412 est majoritairement sorti et le volume de la première chambre de mélange 411 est important, de l'ordre de 4 millilitres. Cette chambre de mélange 411 est remplie par de l'air.

On notera que la tête 213 de l'actionneur 21 ne touche pas, ici, le premier piston 412.

Dans la seconde seringue 42, le deuxième piston 422 est principalement rentré et le volume de la deuxième chambre de mélange 421 est faible, de l'ordre de moins de 1 millilitre. Cette chambre de mélange 421 est remplie par le sclérosant liquide de couleur translucide.

La tête 223 de l'actionneur 22 touche, ici, le deuxième piston 422.

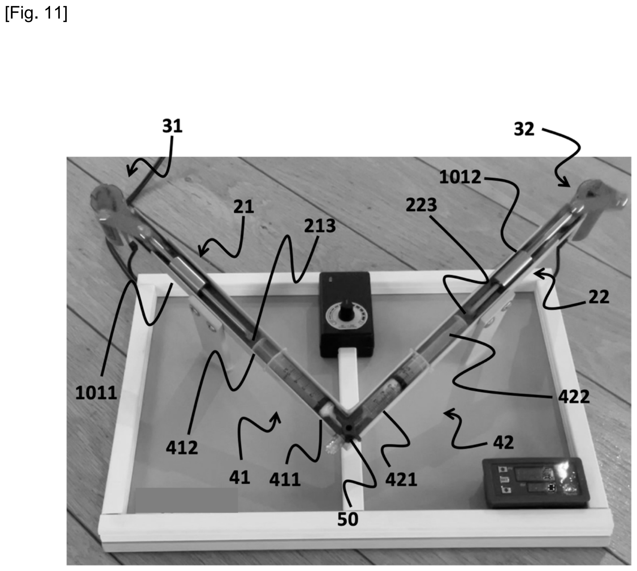

Dans une deuxième étape illustrée à la figure 11, le premier moteur 31 et le deuxième moteur 32 sont en action. La tête 213 de l'actionneur 21 va venir toucher le premier piston 412 pour diminuer le volume de la première chambre de mélange 411. L'air contenue dans cette première chambre de mélange 411 va alors se diriger dans la deuxième chambre de mélange 421 via le connecteur 50. Le deuxième piston 422 n'étant plus bloqué par la tête 223 de l'actionneur 22, le volume de deuxième chambre de mélange 421 s'agrandit. On notera que de la mousse sclérosante (en blanc) commence à sa former à la fois dans la première chambre de mélange 411 et dans la deuxième chambre de mélange 421 du fait de l'excitation des molécules de liquide et d'air dans ces chambres.

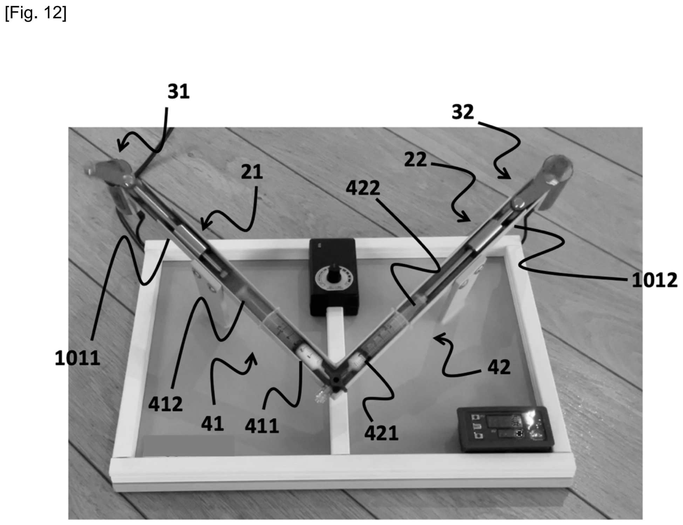

Dans une troisième étape illustrée à la figure 12, la mousse sclérosante est obtenue dans la première chambre de mélange 411 et dans la deuxième chambre de mélange 421 au fur et à mesure des va-et-vient des pistons 412 et 422 contrôlés par le premier actionneur 21 et le deuxième actionneur 22.

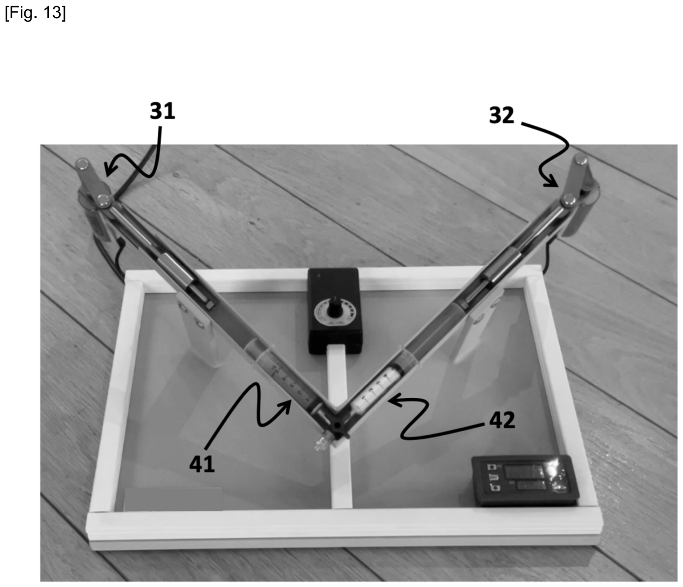

Dans la quatrième étape illustrée à la figure 13, le premier moteur 31 et le deuxième moteur 32 sont mis à l'arrêt. Il est alors possible de recueillir l'ensemble de la mousse sclérosante ainsi formée dans la deuxième seringue 42. Le praticien peut injecter cette mousse dans le corps du patient via cette deuxième seringue 42.

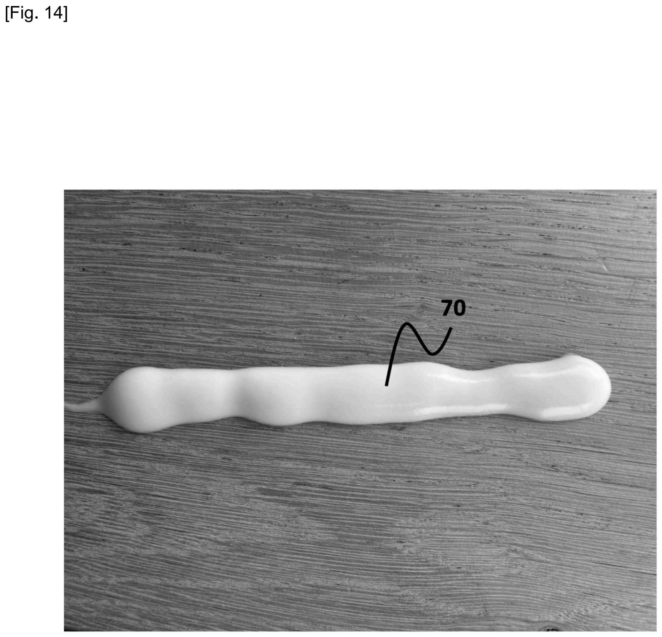

La figure 14 illustre une ligne de mousse sclérosante 70 déposée sur un support via la deuxième seringue 42. Cette mousse présente une certaine compacité. Elle comprend des microbulles de taille homogène, de l'ordre de 100µm.

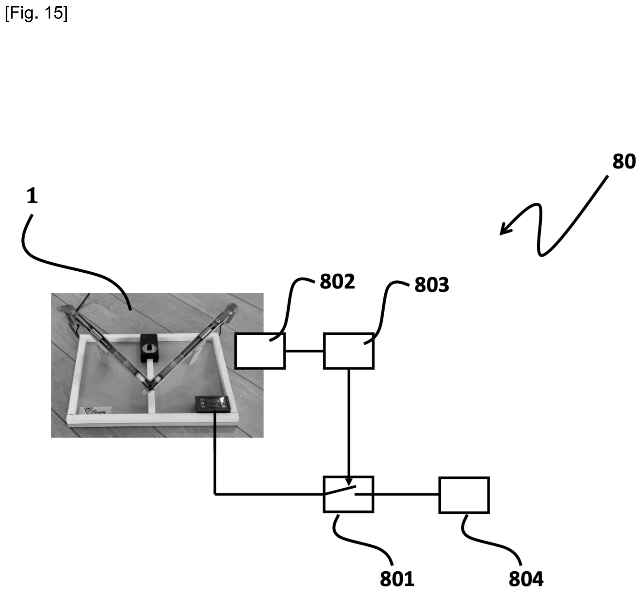

La figure 15 illustre un système 80 de préparation de la mousse sclérosante.

Ce système 80 comprend ici :
- le dispositif 1 de préparation de la mousse sclérosante ;
- un interrupteur d'alimentation 801 ;
- un détecteur 802 ;
- des moyens d'analyse 803.

L'interrupteur d'alimentation 801 est relié à un dispositif d'alimentation électrique 804. L'interrupteur d'alimentation 801 est apte à interrompre l'alimentation électrique du dispositif 1 de préparation de la mousse sclérosante.

Le détecteur 802 est apte à détecter un paramètre de la mousse sclérosante en préparation, tel que la taille des microbulles et leur homogénéité dans la mousse. Ce détecteur 802 est, par exemple, une caméra générant un flux vidéo.

Les moyens d'analyse 803 sont aptes à déterminer si le paramètre détecté de la mousse en préparation est supérieur à un certain seuil. Si ce paramètre est supérieur à ce seuil, il est considéré que la mousse est prête pour une injection et les moyens d'analyse 803 activent l'interrupteur d'alimentation 801 pour stopper l'alimentation du dispositif 1. Les moteurs 31 et 32 sont alors immobilisés.

Dans un mode de réalisation particulier, les moyens d'analyse 803 comprennent un module d'intelligence artificielle adapté pour déduire directement la valeur du paramètre recherché à partir du flux vidéo généré par la caméra.

Dans un mode de réalisation particulier, le volume des seringues peut varier de 2,5 à 10 ml.

## Revendications

1. Dispositif de préparation d'une mousse sclérosante à partir d'un sclérosant liquide et d'un volume d'air, ladite mousse sclérosante étant destinée à être injectée dans un corps d'un patient, ledit dispositif (1) comprenant :
- un châssis (10) comportant une partie (100) en forme de V comprenant un premier bras (1011), un deuxième bras (1012) et une zone de jonction (1013) entre le premier bras (1011) et le deuxième bras (1012), ledit premier bras (1011) étant adapté pour recevoir une première seringue (41), ledit deuxième bras étant adapté pour recevoir une deuxième seringue (52), la zone de jonction (1013) étant adaptée pour recevoir un connecteur (50) disposé entre la première seringue (41) et la deuxième seringue (42) ;
- un premier actionneur (21) disposé dans le premier bras (101), ledit premier actionneur (21) étant destiné à agir sur la première seringue (41) pour modifier un volume d'une première chambre de mélange (411) de ladite première seringue (41) ;
- un deuxième actionneur (22) disposé dans le deuxième bras (1012), ledit deuxième actionneur (22) étant destiné à agir sur la deuxième seringue (42) pour modifier un volume d'une deuxième chambre de mélange (421) de ladite deuxième seringue (42) ;
- un premier moteur (31), disposé à une extrémité du premier bras (101) opposée à la zone de jonction (103), ledit premier moteur (31) étant apte à contrôler le mouvement du premier actionneur (21) ;
- un deuxième moteur (32), disposé à une extrémité du deuxième bras (102) opposée à la zone de jonction (103), ledit deuxième moteur (32) étant apte à contrôler le mouvement du deuxième actionneur (22) ;
- le premier actionneur (21), le deuxième actionneur (22), le premier moteur (31) et le deuxième moteur (32) étant configurés de sorte que lorsqu'ils sont en mouvement, le volume de la première chambre de mélange (411) de la première seringue (41) et le volume de la deuxième chambre de mélange (421) de la deuxième seringue (42) sont modifiés de manière alternée de sorte à permettre un mélange du sclérosant liquide et du volume d'air dans la première seringue (41) et dans la deuxième seringue (42) via le connecteur (50), et dans lequel la partie (100) en forme de V est inclinée par rapport à un plan horizontal (P).

2. Dispositif de préparation selon la revendication 1, dans lequel la partie (100) en forme de V présente un angle interne (α) mesuré entre le premier bras (1011) et le deuxième bras (1012) compris entre 45 degrés et 180 degrés.

3. Dispositif de préparation selon l'une quelconque des revendications 1 à 2, dans lequel le connecteur (50) est un robinet à trois voies.

4. Dispositif de préparation selon l'une quelconque des revendications 1 à 3, dans lequel le premier moteur (31) et le deuxième moteur (32) comprennent respectivement :
- un corps de moteur (311, 321) ;
- un arbre moteur apte à être en rotation par rapport au corps de moteur (311, 321), ledit arbre moteur présentant une extrémité ;
- une première plaque plate allongée (313, 323) présentant une première extrémité proximale (3131, 3231) et une première extrémité distale (3132, 3232), ladite première extrémité proximale (3131, 32231) étant solidaire de l'extrémité de l'arbre moteur ;
- une deuxième plaque plate allongée (314, 324) présentant une deuxième extrémité proximale (3141, 3241) et une deuxième extrémité distale (3142, 3242), ladite deuxième extrémité proximale (3141, 3241) étant fixée avec la première extrémité distale (3132, 3232) de la première plaque plate allongée (313, 323) via une première liaison pivot (P1), ladite deuxième extrémité distale (3142, 3242) étant fixée via une deuxième liaison pivot (P2) avec, respectivement, le premier actionneur (21) ou le deuxième actionneur (22) de sorte à mettre en mouvement ledit premier actionneur (21) ou ledit deuxième actionneur (22) lors de la rotation de l'arbre moteur.

5. Dispositif de préparation selon l'une quelconque des revendications 1 à 4, dans lequel la première plaque plate allongée (313, 323) présente une première longueur entre la première extrémité proximale (3131, 3231) et la première extrémité distale (3132, 3232) et la deuxième plaque plate allongée présente une deuxième longueur entre la deuxième extrémité proximale (3141, 3241) et la deuxième extrémité distale (3142, 3242), ladite deuxième longueur étant supérieure à ladite première longueur.

6. Dispositif de préparation selon l'une quelconque des revendications 1 à 5, dans lequel ledit dispositif comprend un potentiomètre pour gérer la vitesse de déplacement du premier actionneur et du deuxième actionneur.

7. Système de préparation d'une mousse sclérosante à partir d'un sclérosant liquide et d'un volume d'air, ladite mousse sclérosante étant destinée à être injectée dans un corps d'un patient, ledit système 80 comprenant :
- un dispositif (1) de préparation selon l'une quelconque des revendications 1 à 6 ;
- un interrupteur d'alimentation (801) pour interrompre une alimentation électrique du dispositif (1) de préparation ;
- un détecteur (802) d'un paramètre de la mousse sclérosante en préparation ;
- des moyens d'analyse (803), lesdits moyens d'analyse étant aptes à déterminer si le paramètre détecté est supérieur à un certain seuil et à activer l'interrupteur d'alimentation (801) en conséquence.

8. Système de préparation selon la revendication 7, dans lequel les moyens d'analyse (803) comprennent un module d'intelligence artificielle.

9. Mousse sclérosante destinée à être injectée dans un corps d'un patient, ladite mousse sclérosante étant préparée à l'aide d'un dispositif de préparation selon l'une quelconque des revendications 1 à 6.
